# EUROPÄISCHE PATENTSCHRIFT

(11) **EP 1 265 874 B1**
(45) Veröffentlichungstag und Bekanntmachung des Hinweises auf die Patenterteilung: **01.06.2005**
(21) Anmeldenummer: 01953631.7
(22) Anmeldetag: 23.01.2001
(51) Int. Cl.: C07D 239/94

(54) **VERFAHREN ZUR HERSTELLUNG VON (3-CHLOR-4-FLUOR-PHENYL)-[7-(3-MORPHOLIN-4-YL-PROPOXY)-6-NITRO-CHINAZOLIN-4-YL]-AMIN BZW. (3-CHLOR-4-FLUOR-PHENYL)-[7-(3-MORPHOLIN-4-YL-PROPOXY)-6-AMINO -CHINAZOLIN-4-YL]-AMIN**
METHOD FOR THE PRODUCTION OF (3-CHLORO-4-FLUOROPHENYL)-[7-(3-MORPHOLIN-4-YLPROPOXY)-6-NITROQUINAZOLIN-4-YL]-AMINE OR (3-CHLORO-4-FLUOROPHENYL)-[7-(3-MORPHOLIN-4-YL-PROPOXY)-6-AMINOQUINAZOLIN-4-YL]-AMINE
PROCEDE DE PREPARATION DE (3-CHLORO-4-FLUOROPHENYL)-[7-(3-MORPHOLIN-4-YLPROPOXY)-6-NITROQUINAZOLIN-4-YL]-AMINE OU DE (3-CHLORO-4-FLUOROPHENYL)-[7-(3-MORPHOLIN-4-YLPROPOXY)-6-AMINOQUINAZOLIN-4-YL]-AMINE

(30) Priorität: 26.02.2000 DE 10009267
(43) Veröffentlichungstag der Anmeldung: 18.12.2002
(73) Patentinhaber: GÖDECKE GMBH, 76139 Karlsruhe (DE)
(72) Erfinder: BARTH, Hubert, Dr., 79312 Emmendingen (DE); STEINER, Klaus, Dr., 79312 Emmendingen (DE); SCHNEIDER, Simon, 79249 Merzhausen (DE)
(74) Vertreter: Tesch, Rudolf, Dr.
(86) Internationale Anmeldenummer: PCT/EP2001/000695
(87) Internationale Veröffentlichungsnummer: WO 2001/062743

(56) Entgegenhaltungen:
- WO-A-97/38983

## Beschreibung

Bei (3-Chlor-4-fluor-phenyl)-[7-(3-morpholin-4-yl-propoxy)-6-nitro-chinazolin-4-yl]-amin (I) 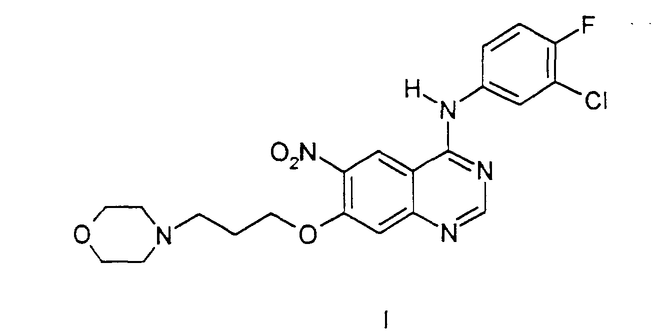 handelt es sich um eine Schlüsselverbindung zur Darstellung des Dihydrochlorids von N-[4-[(3-Chlor-4-fluorphenyl)amino]-7-[3-(4-morpholinyl)propoxy]-chinazolinyl]-2-propenamid (II), 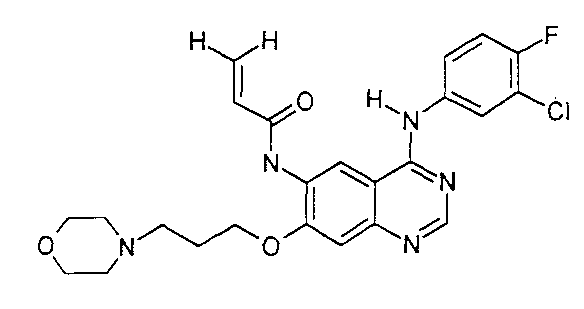 einem Vertreter einer neuen Klasse von hoch wirksamen irreversiblen Tyrosin Kinase Inhibitoren des EGFR's (Epidermal Growth Factor Receptor), die zur Behandlung verschiedener Tumoren eingesetzt werden sollen, was beispielsweise in WO 97/38983 beschrieben ist.

Bei den o. g. irreversiblen Tyrosin Kinase Inhibitoren des EGFR handelt es sich um substituierte 4-Anilinochinazolinderivate, die bisher z.T. nur in einer bis zu 12-stufigen Synthese hergestellt werden konnten. Die ursprünglichen Syntheseschritte sind beschrieben in J. Med. Chem. 1996, 39, 918-928 und WO 97/38983.

Ausgangsmaterial für die bisherige Synthese war isomerenreines 7-Fluor-6-nitrochinazolin-4(3H)-on (III), 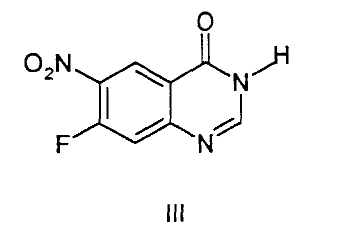 das ohne Lösungsmittel mit einem 55 molaren Überschuß an Thionylchlorid unter Zusatz von katalytischen Mengen an DMF zu 4-Chlor-7-fluor-6-nitrochinazolin (IV) 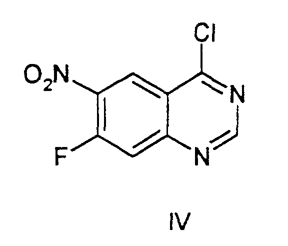 umgesetzt wurde. Nach Abdestillieren des Thionylchlorids wurde das als Rohprodukt anfallende 4-Chlor-7-fluor-6-nitrochinazolin portionsweise zu einer Lösung von 1 Äquivalent 3-Chlor-4-fluoranilin und 2 Äquivalenten des hoch toxischen N,N-Dimethylanilin in 2-Propanol umgesetzt. Nach 6-stündigem Rühren bei 25 °C wurde nach einer wäßrigen Aufarbeitung 4-(3-Chlor-4-fluoranilino)-7-fluor-6-nitrochinazolin (V) 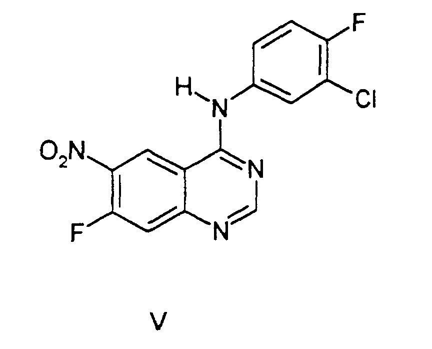 in ca. 90 %iger Ausbeute als Rohprodukt erhalten.

Zu einer Suspension von 1 Äquivalent 4-(3-Chlor-4-fluoranilino)-7-fluor-6-nitrochinazolin (V) und 1,5 Äquivalenten 3-(4-morpholino)propan-1-ol (VI) 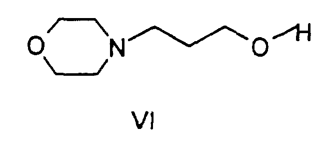 in Dimethylsulfoxid (DMSO) wurde eine Lösung von 3 Äquivalenten Kalium-trimethylsilanolat in DMSO zugetropft und das Reaktionsgemisch ca. 6 Stunden gerührt. Nach einer wäßrigen Aufarbeitung wurde (3-Chlor-4-fluor-phenyl)-[7-(3-morpholin-4-yl-propoxy)-6-nitro-chinazolin-4-yl]-amin (I) in ca. 89 %iger Ausbeute erhalten.

Dieser Syntheseschritt erwies sich als besonders problematisch, da bei Ansatzvergrößerungen nur sehr schwankende Ausbeuten und zum Teil auch deutlich geringere Ausbeuten als beschrieben erhalten wurden.

Reaktionen von 4-(3-Chlor-4-fluoranilino)-7-fluor-6-nitrochinazolin (V) und 3-(4-Morpholino)propan-1-ol (VI) und festem Natriumhydrid in THF analog dem in WO 97/38983 beschrieben Verfahren führten ebenfalls nur zu unbefriedigenden Ergebnissen.

Das erhaltene (3-Chlor-4-fluor-phenyl)-[7-(3-morpholin-4-yl-propoxy)-6-nitrochinazolin-4-yl]-amin (I) wurde anschließend unter anderem über Raney-Nickel in THF als Lösungsmittel zu (3-Chlor-4-fluor-phenyl)-[7-(3-morpholin-4-yl-propoxy)-6-amino-chinazolin-4-yl]-amin (VII) 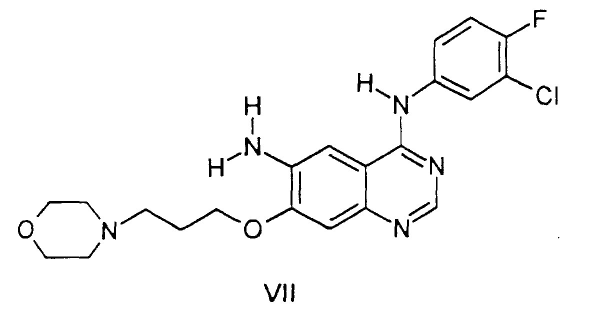 hydriert, das dann weiter zu (II) bzw. dessen Dihydrochlorid-trihydrat umgesetzt wurde.

Aufgabe der Erfindung war es daher, ein ökonomisches und technisch durchführbares Verfahren zur Herstellung der o. g. Schlüsselverbindungen (I) bzw. (VII) zu entwickeln. Hierbei sollten:
1.) die Menge des verwendeten Thionylchlorids reduziert werden,
2.) auf den Einsatz des hoch toxischen N,N-Dimethylanilins verzichtet werden,
3.) DMSO durch ein billigeres Lösungsmittel ersetzt werden,
4.) Kalium-trimethylsilanolat oder Natriumhydrid ersetzt werden,
5.) die Ausbeuten des Umsatzes von (I) und (VI) konstant gut sein,
6.) und auf eine jeweilige Isolierung der Einzelverbindungen (IV), (V), und ggf. von (I) verzichtet werden.

Gegenstand der Erfindung ist daher die Zusammenfassung der einzelnen Reaktionsschritte zu einer Eintopf-Reaktion.

Überraschenderweise wurde gefunden, daß sich die Chlorierungsreaktion von (III) mit Thionylchlorid zu (IV), die Umsetzung des gebildeten Chlorids (IV) mit 3-Chlor-4-fluoranilin zu (V) und die Folgereaktion von (V) mit 3-Morpholin-4-ylpropan-1-ol (VI) zu der Schlüsselverbindung (I) in ausgezeichneter Gesamtausbeute, ohne Isolierung der Zwischenverbindungen, zu einer ,Eintopf-Reaktion' mit 3 Reaktionsschritten zusammenfassen lassen, wie im Reaktionsschema (Fig. 1) dargestellt.

Weiterhin wurde überraschenderweise gefunden, daß es auch nicht notwendig ist, die Verbindung (I) zu isolieren, sondern daß das bei der o. g. ,Eintopfreaktion' erhaltene Reaktionsgemisch direkt für die Hydrierung zu (VII) eingesetzt werden kann, d.h. zu einer Eintopfreaktion mit 4 Reaktionsschritten.

Weiterhin konnte bei der Chlorierungsreaktion der 55 molare Überschuß an Thionylchlorid auf einen 11,5 molaren Überschuß reduziert werden. Nach Abdestillieren des Thionylchlorids wird restliches Thionylchlorid mehrfach azeotrop mit Toluol abdestilliert. Bei der letzten Toluoldestillation muß das Toluol nur soweit abdestilliert werden, daß ein noch gut rührbarer grobkristalliner Rückstand zurück bleibt. Das so gebildete Chlorid (IV) ist sehr rein und wird direkt mit einer Tetrahydrofuran/tert.-Butanol-Mischung (7:3) versetzt. Für den weiteren Reaktionsverlauf ist der Einsatz dieses Tetrahydrofuran/tert.-Butanol-Gemisches von größter Wichtigkeit. Überraschenderweise wurde gefunden, daß der der Reaktionsmischung beigesetzte tert.-Butylalkohol die gewünschte Substitutions- reaktion katalysiert.

Bei der nachfolgenden Reaktion des Chlorids (IV) mit 3-Chlor-4-fluoranilin konnte das als Säurefänger ursprünglich eingesetzte hochtoxische N,N-Dimethylanilin durch das im 3. Reaktionsschritt notwendige Amin 3-Morpholin-4-yl-propan-1-ol (VI) ersetzt werden. Nach ca. 24-std. Rühren bei Raumtemperatur war das Chlorid (IV) vollständig zu dem Anilinderivat (V) umgesetzt. Überraschenderweise reagierte bei dieser Reaktion nicht die Alkoholgruppe der zugesetzten Base. Die hierbei entstehende gelbe bis orangefarbene Suspension wurde direkt mit einer Kalium-tert.-Butylat/THF-Lösung versetzt. Hierbei wurde das im vorherigen Reaktionsschritt als Säurefänger eingesetzte 3-Morpholin-4-yl-propan-1-ol (VI) wieder in die freie Base überführt, die dann in Gegenwart von Kalium-tert.-Butylat sofort in der gewünschten Weise mit dem im Reaktionsgemisch schon vorliegendem Anilinderivat (VI) zu dem gewünschten (I) weiter reagierte, das nach Quenchen mit einem Eis/Ethanol/Salzsäuregemisch in einer überraschend guten Gesamtausbeute von ca. 95 % mit einer ebenfalls überraschend guten Reinheit von > 98 % erhalten wurde.

Dieser sehr gute Reaktionsverlauf war um so überraschender, da der im Reaktionsgemisch vorliegende und sich auch neu bildende tert.-Butylalkohol nicht in analoger Weise wie 3-Morpholin-4-yl-propan-1-ol (VI) mit (V) zu dem entsprechenden tert.-Butylether reagierte.

Weiterhin war überraschend, daß das für solche Substitutionsreaktionen eingesetzte NaH (WO 97/38983; J. Med.Chem.; 35; 14; 1992; 2617 - 2626; J. Amer. Chem. Soc. 76, 1954, 3032, Heterocycles, 22; 1; 1984; 73-78), Natriumamid (J. Org. Chem. 59; 21; 1994: 6194 - 6199) oder Kalium trimethylsilonat durch das unproblematisch handhabbare Kalium-tert.-Butylat ersetzt werden konnte.

Überraschender Weise war das im Reaktionsgemisch anfallende Produkt (I) von einer derart hohen Reinheit, daß die Reaktionslösung ohne Isolierung von (I) direkt für die nachfolgende Hydrierung eingesetzt werden konnte.

### Beispiel 1:

### Eintopfreaktion zur Herstellung von (3-Chlor-4-fluor-phenyl)-[7-(3-morpholin-4-yl-propoxy)-6-nitro-chinazolin-4-yl]-amin (I).

150 g 7-Fluor-6-nitrochinazolin-4(3H)-on (III) werden in 600 mL Thionylchlorid suspendiert und nach Zugabe von 6 mL DMF während 24 Std. unter Rückfluß gekocht. Hierbei entsteht eine klare Lösung. Im Vakuum werden ca. 350 mL Thionylchlorid abdestilliert. Die entstehende grob-kristalline Suspension wird mit ca. 600 mL Toluol versetzt. Im Vakuum werden ca. 800 mL abdestilliert. Diese Destillation wird noch 3 mal mit jeweils 600 mL frischem Toluol wiederholt. Bei der letzten Destillation wird das Toluol so weit wie möglich abdestilliert. Es entsteht eine grob-kristalline Suspension, die jederzeit gut rührbar bleibt.

Der nahezu trockene Rückstand wird mit 1,2 L einer Tetrahydrofuran/tert.-Butanol-Mischung (7:3) versetzt. Die entstehende Suspension wird auf ca. 10°C gekühlt. Unter gutem Rühren und Kühlen wird eine Lösung aus 114 g 3-Chlor-4-fluoranilin und 258 g 3-Morpholin-4-yl-propan-1-ol (VI) in 300 mL THF/tert.-Butanol (7:3) während ca. 20 Min. so zugetropft, daß die Temperatur im Reaktor zwischen 10°C und 15°C bleibt. Die anfangs gelbliche Suspension wird im Verlauf des Zutropfens dünner und verfärbt sich nach orange.

Das Reaktionsgemisch läßt man langsam auf Raumtemperatur kommen und rührt anschließend mind. 24 Std. bei Raumtemperatur nach.

Zu der gelb-orangen Suspension wird unter gutem Rühren und leichter Kühlung während ca. 20 Min. eine Lösung von 324 g Kalium-tert.-Butylat in 1,86 L Tetrahydrofuran so zugetropft, daß die Temperatur im Reaktor zwischen 15°C und 20°C bleibt. Nach Zugabe von ca. 1/3 der Kalium-tert.-Butylat/THF-Lösung verfärbt sich das gesamte Reaktionsgemisch dunkelrot.

Nach ca. 30 minütigen Nachrühren wird das Reaktionsgemisch sofort in ein Gemisch aus 5,4 kg Eis, 6,0 L Ethanol und 1,8 L Salzsäure eingerührt (pH der Lösung ca. 8). Hierbei entsteht zunächst eine gelb-orange Lösung. Nach kurzem Rühren kristallisiert ein gelbes Produkt aus. Die entstehende Suspension wird ca. 5 Std. bei ca. 0°C nachgerührt und anschließend abgesaugt. Der Nutschkuchen wird 2 mal mit jeweils 500 mL eiskaltem Ethanol nachgewaschen.

Das Produkt wird im Umlufttrockenschrank zunächst bei 40°C vorgetrocknet und anschließend bei 60°C bis zur Gewichtskonstanz nachgetrocknet. (Ausbeute: 316,5 g = 95,5 %; HPLC-Reinheit: 98,48 rel%; H₂O (K.F.): 3,69 %; Fp.: 257°C).

### Beispiel 2:

### Eintopfreaktion zur Herstellung von (3-Chlor-4-fluor-phenyl)-[7-(3-morpholin-4-yl-propoxy)-6-amino-chinazolin-4-yl]-amin (VII).

20 g 7-Fluor-6-nitrochinazolin-4(3H)-on (III) werden in 80 mL Thionylchlorid suspendiert und nach Zugabe von 20 Tropfen DMF während 24 Std. unter Rückfluß gekocht. Hierbei entsteht eine klare Lösung. Im Vakuum werden ca. 60 mL Thionylchlorid abdestilliert. Die entstehende grob-kristalline Suspension wird mit ca. 60 mL Toluol versetzt. Im Vakuum werden ca. 60 mL abdestilliert. Diese Destillation wird noch 3 mal mit jeweils 60 mL frischem Toluol wiederholt. Bei der letzten Destillation wird das Toluol so weit wie möglich abdestilliert. Es entsteht eine grob-kristalline Suspension, die jederzeit gut rührbar bleibt.

Der nahezu trockene Rückstand wird mit 160 mL einer Tetrahydrofuran/tert.-Butanol-Mischung (7:3) versetzt. Die entstehende Suspension wird auf ca. 10°C gekühlt. Unter gutem Rühren und Kühlen wird eine Lösung aus 15,2 g 3-Chlor-4-fluoranilin und 34,4 g 3-Morpholin-4-yl-propan-1-ol (VI) in 40 mL THF/tert.-Butanol (7:3) während ca. 20 Min. so zugetropft, daß die Temperatur im Reaktor zwischen 10°C und 15°C bleibt. Die anfangs gelbliche Suspension wird im Verlauf des Zutropfens dünner und verfärbt sich nach orange.

Das Reaktionsgemisch läßt man langsam auf Raumtemperatur kommen und rührt anschließend mind. 24 Std. bei Raumtemperatur nach.

Zu der gelb-orangen Suspension wird unter gutem Rühren und leichter Kühlung während ca. 20 Min. eine Lösung von 43,2 g Kalium-tert.-Butylat in 250 mL Tetrahydrofuran so zugetropft, daß die Temperatur im Reaktor zwischen 15°C und 20°C bleibt. Nach Zugabe von ca. 1/3 der Kalium-tert.-Butylat/THF-Lösung verfärbt sich das gesamte Reaktionsgemisch dunkelrot.

Nach ca. 30 minütigen Nachrühren wird das Reaktionsgemisch bei 0°C - 5°C mit einer Mischung aus 20 mL Salzsäure und 30 mL Wasser versetzt und mit weiteren 200 mL THF verdünnt. Nach 20 minütigem Rühren im Eisbad wird die Reaktionsmischung über 50 g Celite klarfiltriert. Der Filterkuchen wird mit 100 mL THF ausgewaschen. Das Filtrat wird mit 31 g Raney-Nickel versetzt und bei Raumtemperatur 3 Std. bei 3,5 bar mit Wasserstoff hydriert. Nach Absaugen des Katalysators wird das Filtrat bis zur Trockene eingeengt und der Rückstand mit 80 mL Ethanol bei ca. 2°C verührt. Das ausgefallene Produkt wird abgesaugt und mit wenig kaltem Ethanol gewaschen. Nach Trocknen im Umluftrockenschrank bei 60°C werden 32,1 g (77,7 %) Produkt erhalten.

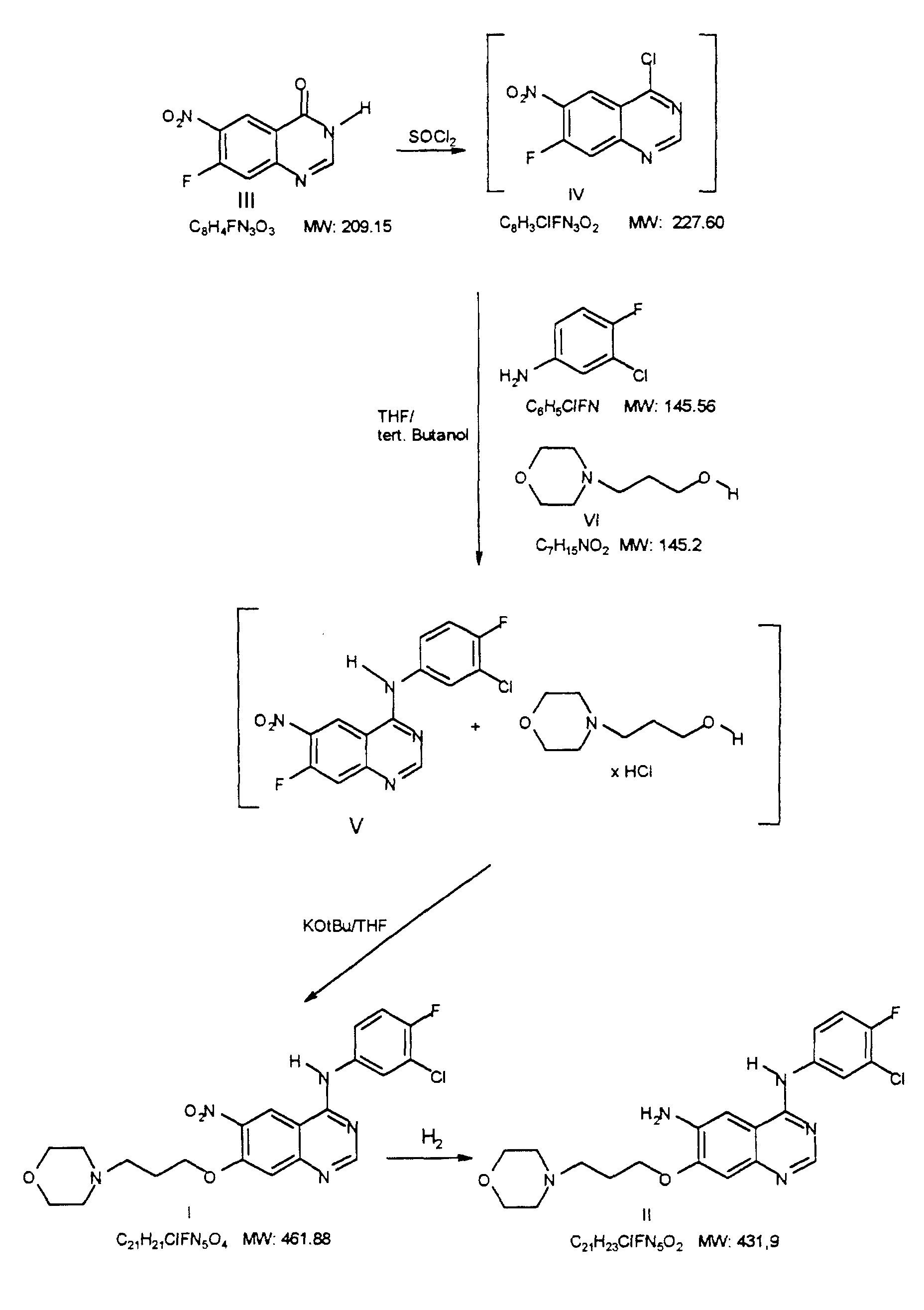

## Patentansprüche

1. Verfahren zur Herstellung von (3-Chlor-4-fluor-phenyl)-[7-(3-morpholin-4-yl-propoxy)-6-nitro-chinazolin-4-yl]-amin (I) 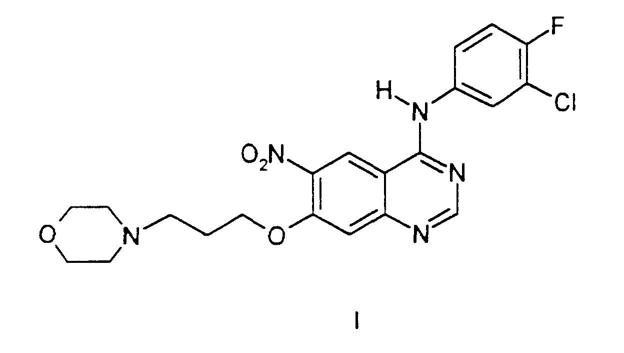 bzw. von (3-Chlor-4-fluor-phenyl)-[7-(3-morpholin-4-yl-propoxy)-6-amino-chinazolin-4-yl]-amin (VII) 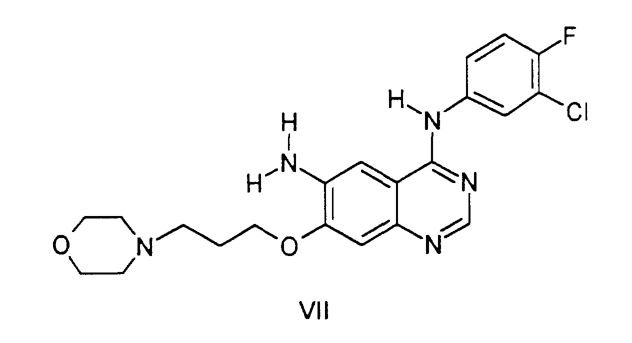 **dadurch gekennzeichnet, daß** man in einer Eintopf-Reaktion in 3 bzw. 4 Reaktionsschritten zunächst 7-Fluor-6-nitrochinazolin-4(3H)-on (III), 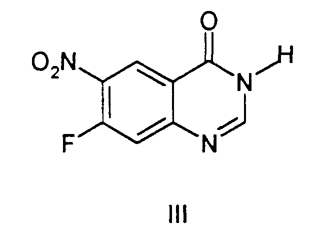 mit Thionylchlorid zu 4-Chlor-7-fluor-6-nitrochinazolin (IV), 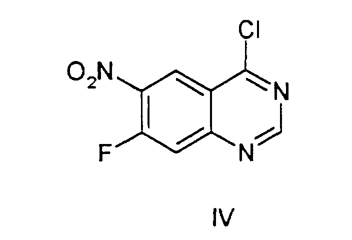 dieses mit 3-Chlor-4-fluoranilin zu 4-(3-Chlor-4-fluoranilino)-7-fluoro-6-nitrochinazolin (V), 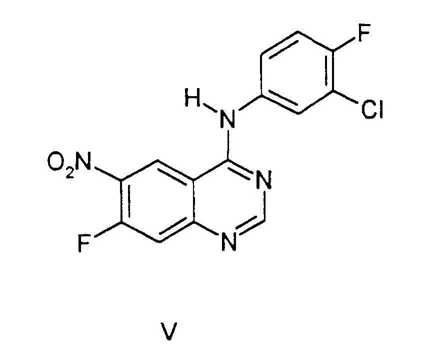 das anschließend mit 3-Morpholin-4-yl-propan-1-ol (VI) 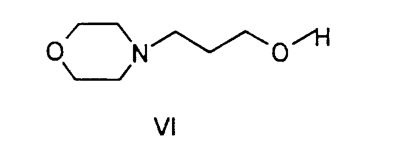 zu (I) umsetzt und gegebenfalls (I) in der Reaktionslösung direkt zu (VII) hydriert.

## Claims

1. Method for the production of (3-chloro-4-fluorophenyl)-[7-(3-morpholin-4-yl-propoxy)-6-nitroquinazoline-4-yl]-amine (I) 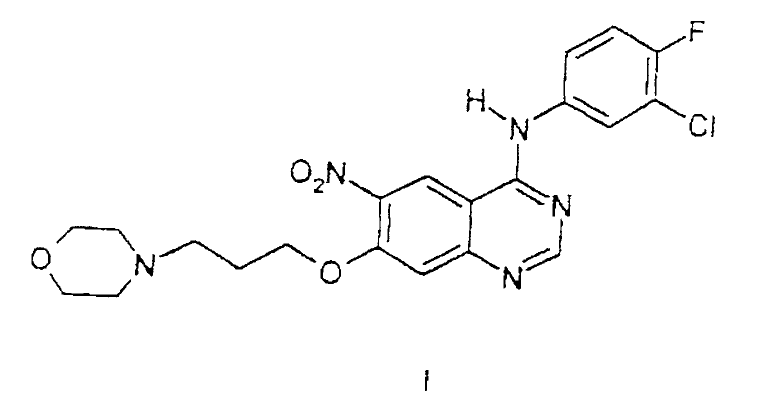 or of (3-chloro-4-fluorophenyl)-[7-(3-morpholine-4-yl-propoxy)-6-aminoquinazoline-4-yl]-amine (VII) 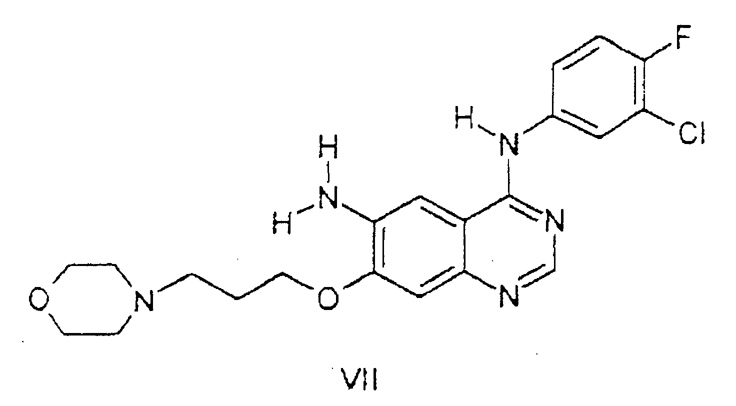 **characterised in that** a one pot reaction with 3 or 4 steps is first used for the reaction of 7-fluoro-6-nitroquinazoline-4(3H)-on (III), 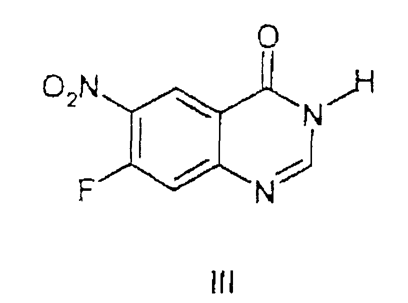 with thionyl chloride into 4-chloro-7-fluoro-6-nitroquinazoline (IV), 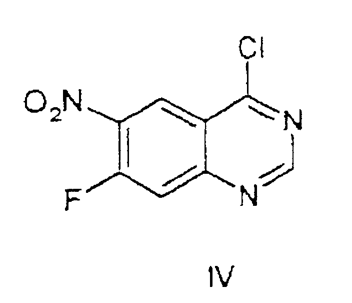 which is reacted with 3-chloro-4-fluoroaniline into 4-(3-chloro-4-fluoroanilino)-7-fluoro-6-nitroquinazoline (V) 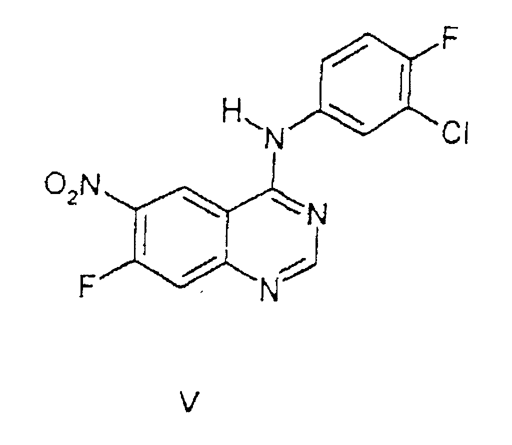 that subsequently reacts with 3-morpholin-4-yl-propane-1-ol (VI) 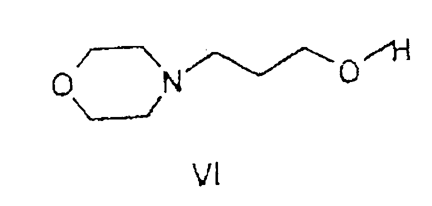 into (I) and, where appropriate, directly hydrogenates (I) in the reaction solution to produce (VII).

## Revendications

1. Procédé pour la préparation de (3-chloro-4-fluoro-phényl)-[7-(3-morpholin-4-yl-propoxy)-6-nitroquinazoline-4-yl]amine (1) 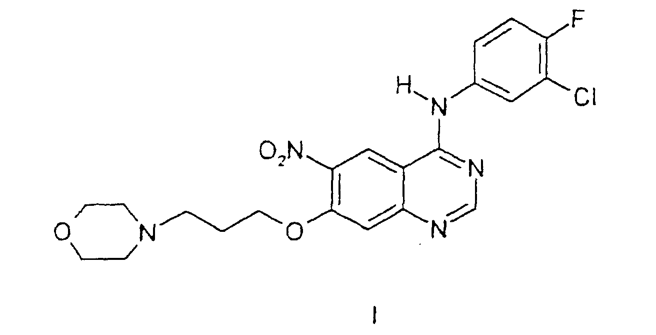 respectivement de (3-chloro-4-fluoro-phényl)-[7-(3-morpholin-4-yl-propoxy)-6-amino-quinazolin-4-yl]-amine (VII) 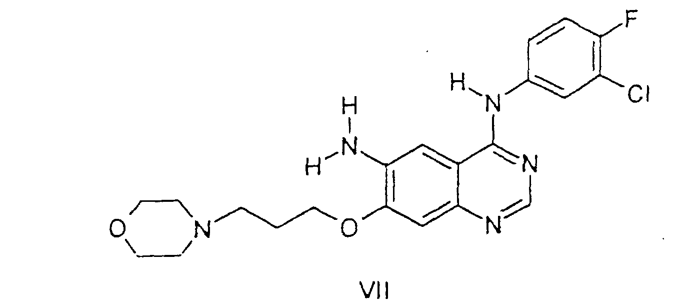 **caractérisé en ce qu'**on transforme dans une réaction se déroulant dans un pot, en 3 respectivement 4 étapes de réaction, d'abord de la 7-fluoro-6-nitroquinazolin-4(3H)-one (III), 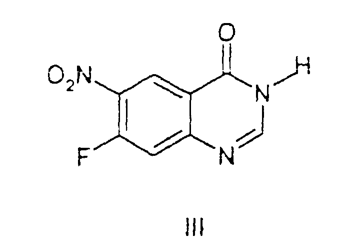 avec du chlorure de thionyle en 4-chloro-7-fluoro-6-nitroquinazoline (IV) 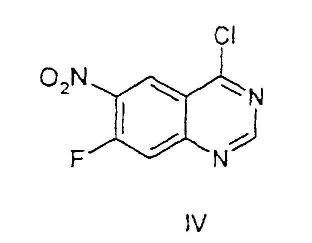 on transforme celle-ci avec de la 3-chloro-4-fluoroaniline en 4-(3-chloro-4-fluoroanilino)-7-fluoro-6-nitroquinazoline (V), 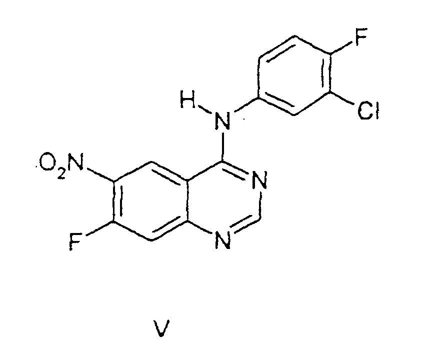 qui est ensuite transformée avec du 3-morpholin-4-yl-propan-1-ol (VI) 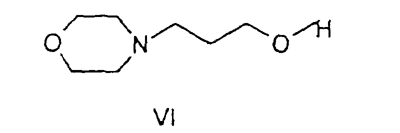 en (I) et on hydrogène le cas échéant (I) dans la solution réactionnelle directement en (VII).
